# EUROPEAN PATENT SPECIFICATION

(11) **EP 3 545 840 B1**
(45) Date of publication and mention of the grant of the patent: **04.11.2020**
(21) Application number: 17873841.5
(22) Date of filing: 07.11.2017
(51) Int. Cl.: A61B 5/151, A61B 5/15

(54) **PUNCTURE NEEDLE CARTRIDGE, AND PUNCTURE INSTRUMENT FOR ALLOWING SAME TO BE MOUNTED THERETO**
PUNKTIONSNADELKARTUSCHE UND PUNKTIONSINSTRUMENT ZUR ERMÖGLICHUNG VON DEREN BEFESTIGUNG DARAN
CARTOUCHE D'AIGUILLE À PONCTION, ET INSTRUMENT DE PONCTION PERMETTANT SON MONTAGE SUR LADITE CARTOUCHE

(30) Priority: 22.11.2016 JP 2016226514; 03.02.2017 JP 2017018233
(43) Date of publication of application: 02.10.2019
(73) Proprietor: PHC Holdings Corporation, Tokyo 105-8433 (JP)
(72) Inventor: YASUI, Shinichi, Toon-shi, Ehime 791-0395 (JP)
(74) Representative: Vigand, Philippe
(86) International application number: PCT/JP2017/040124
(87) International publication number: WO 2018/096923

(56) References cited:
- WO-A1-2009/110247
- JP-A- 2016 101 476

## Description

### TECHNICAL FIELD

The present invention relates to a puncture needle cartridge for puncturing a human body with a needle in order to measure the blood glucose level, for example, and to a puncture instrument to which this puncture needle cartridge is attached.

### BACKGROUND ART

A puncture needle cartridge is attached to a puncture instrument, and then a puncture needle cover is removed, after which a puncture operation is performed toward the puncture site.

That is, this type of puncture needle cartridge comprises a lancet body having a connector to the puncture instrument on the rear end side, a puncture needle cover on the front end side, and a breakable component in the middle section; a puncture needle that is embedded in this lancet body across the breakable component of the lancet body; and a lancet case that is provided to the outer periphery of the lancet body (see, for example, Patent Literature 1 below).

### CITATION LIST

### PATENT LITERATURE

Patent Literature 1: JP-A 2004-33440
Patent Literature 2: WO 2007/129757 pamphlet
Patent Literature 3: WO 2014/083783 pamphlet

JP 2016 101476 A discloses a puncture needle cartridge attached to a puncture container, the puncture needle cartridge being provided with: a lancet body having a puncture needle, a connection part provided on the opposite side as the puncture needle side and connected to the puncture container, a puncture needle cover part covering the tip of the puncture needle, a breakable part for separating the puncture needle cover part, a guide protrusion provided on the outer peripheral surface of the puncture needle cover, and a sliding protrusion provided closer to the connection part than the breakable part; and a lancet case having a spiral guide part that slides in an engaged state with the sliding protrusion while rotating, and a sliding groove that moves in an engaged state with the sliding protrusion, the lancet case being attached to the outer periphery of a middle part of the lancet body while the breakable part is in an unbroken state.

### SUMMARY

In the above conventional example, when the puncture needle cartridge is attached to the puncture instrument, the breakable component is broken by turning the rear side of the lancet body constituting the puncture needle cartridge past the breakable component with the puncture instrument.

In such a configuration, since a turning mechanism for turning the lancet body of the puncture needle cartridge is provided to the puncture instrument, depending on the state in which the puncture needle cartridge is attached to the puncture instrument, there is a risk that this turning mechanism will not operate properly. As a result, a problem with this conventional configuration was that the breakable component could not be broken and the puncture needle cartridge could not be properly attached to the puncture instrument.

In view of this, it is an object of the present invention to allow a puncture needle cartridge to be properly attached to a puncture instrument.

To achieve this object, the puncture needle cartridge of the present invention is a puncture needle cartridge that is attached to a puncture instrument, and comprises a columnar lancet body and a tubular lancet case. The lancet body includes a puncture needle; a connector that is connected to the puncture instrument on a side of a second end, which is an opposite side from a first end at which puncture is performed with the puncture needle; a puncture needle cover that is provided on a side of the first end side and covers a tip portion of the puncture needle before use; a breakable component that is provided to a middle section between the first end and the second end, is broken when performing puncturing, and separates the puncture needle cover and exposes the tip portion of the puncture needle; guide protrusions that are provided to the outer peripheral face of the puncture needle cover on the side of the first end of the breakable component; and sliding protrusions that are provided closer to the side of the second end than the breakable component. The lancet case has spiral guide components formed from the side of the first end toward the side of the second end, for imparting a rotational force in a specific direction to the lancet case when there is relative movement in the puncture direction in a state in which the guide protrusions are engaged; and a sliding groove for guiding movement in the puncture direction in a state in which the sliding protrusions are engaged, the lancet case being mounted to an outer periphery of the middle section of the lancet body in a state in which the breakable component has not been broken. The puncture needle cover further has stoppers that restrict sliding to the side of the second end from a state in which the lancet case is mounted to the middle section.

### (EFFECTS)

With the puncture needle cartridge disclosed herein, the puncture needle cartridge itself has a turning mechanism such that when the lancet case attached to the outer periphery of the middle section is slid to the puncture side (first end side) as a result of the attachment of the puncture needle cartridge to a puncture instrument, a rotational force is generated in the reverse direction at the connector ahead of and behind the breakable component, and this force breaks the breakable component. Therefore, the turning mechanism can be operated and the breakable component can be broken without being affected by the state in which the puncture needle cartridge is attached to the puncture instrument.

In order to operate the turning mechanism properly, stoppers are provided to the puncture needle cover for restricting the sliding of the lancet case from the middle section to the rear end side (second end side). Therefore, the lancet case slides only from the outer periphery of the middle section to the puncture side (first end side), so the turning mechanism can be properly operation and the breakable component broken by turning the lancet case only in a specific direction.

As a result, the puncture needle cartridge can be properly attached to the puncture instrument.

### BRIEF DESCRIPTION OF DRAWINGS

FIG. 1 is an oblique view showing a usage state of a puncture needle cartridge according to Embodiment 1 of the present invention;
FIG. 2 is an exploded oblique view of the puncture needle cartridge in FIG. 1;
FIG. 3 is an oblique view of the puncture needle cartridge in FIG. 1;
FIG. 4 is an oblique view of the puncture needle cartridge in FIG. 1;
FIG. 5A is a cross section of the puncture needle cartridge in FIG. 1 along A plane in FIG. 3, and FIG. 5B is a developed view of the main parts of the puncture needle cartridge in FIG. 1;
FIG. 6 is an exploded oblique view of a puncture instrument to which the puncture needle cartridge in FIG. 1 has been attached;
FIG. 7 is a cross section of the puncture instrument in FIG. 6;
FIG. 8 is a cross section of the puncture instrument in FIG. 6;
FIG. 9 is a cross section of the puncture instrument in FIG. 6;
FIG. 10 is a cross section of the puncture instrument in FIG. 6;
FIG. 11 is a cross section of the puncture instrument in FIG. 6;
FIG. 12 is a cross section of the puncture instrument in FIG. 6;
FIG. 13 is a cross section of the puncture instrument in FIG. 6;
FIG. 14 is a cross section of the puncture instrument in FIG. 6;
FIG. 15 is an oblique view of the puncture needle cartridge in FIG. 1;
FIGS. 16A, 16B, and 16C are diagrams showing the usage states of the puncture needle cartridge according to Embodiment 1 of the present invention;
FIG. 17A is an oblique view of the puncture needle cartridge according to Embodiment 1 of the present invention, and FIG. 17B is a side view thereof; and
FIG. 18 is an exploded oblique view of the puncture needle cartridge shown in FIG. 17A, etc.

### DESCRIPTION OF EMBODIMENTS

Embodiments will now be described in detail with reference to the drawings as needed. However, unnecessarily detailed description may be omitted. For example, detailed description of already known facts or redundant description of components that are substantially the same may be omitted.

This is to avoid unnecessary repetition in the following description, and facilitate an understanding on the part of a person skilled in the art.

The inventors have provided the appended drawings and the following description so that a person skilled in the art might fully understand this disclosure, but does not intend for these to limit what is discussed in the patent claims.

Embodiments of the present invention will now be described.

### Embodiment 1

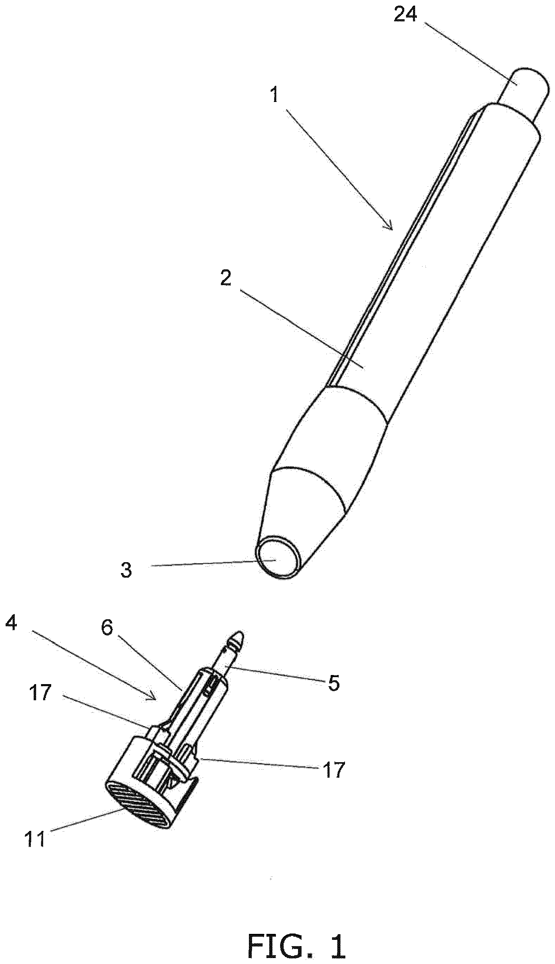

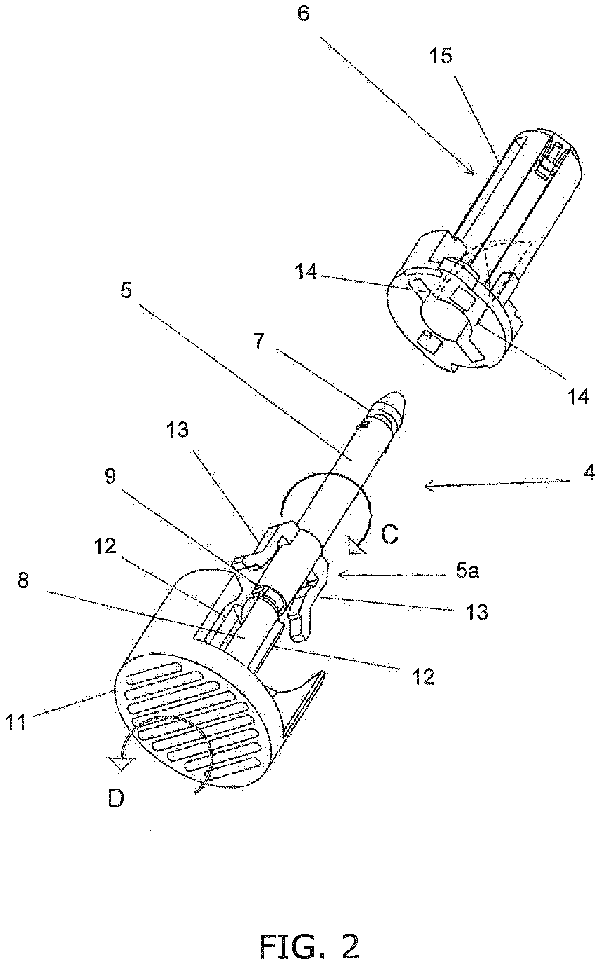

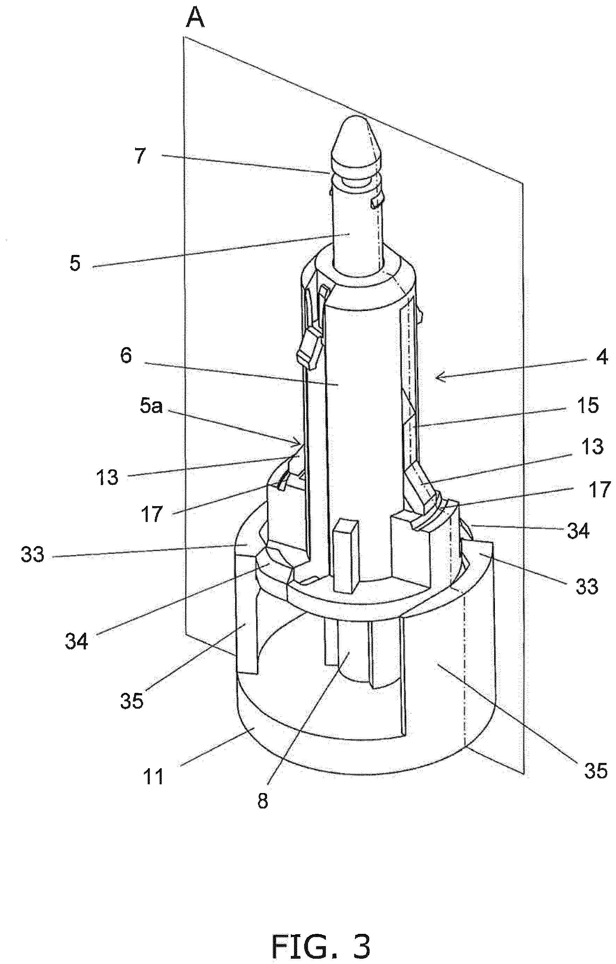

FIG. 1 shows a puncture instrument 1. The puncture instrument 1 is used to insert a needle into a human body and extract blood in order to measure the blood glucose level, for example. The puncture instrument 1 performs puncturing in a state in which the puncture needle cartridge 4 shown in FIGS. 2 and 3 has been attached to the front end opening 3 of a long, tubular main body case 2.

In the following description, "front" means the side on which puncturing is performed by the puncture needle 10 (the first end side), and "rear" means the opposite side (means the second end side).

First, the basic configuration and operation of the puncture needle cartridge 4 will be described.

FIG. 2 is an exploded oblique view of the puncture needle cartridge 4 in this embodiment. The puncture needle cartridge 4 has a substantially circular columnar lancet body 5 made of plastic, and a substantially circular cylindrical lancet case 6 made of plastic.

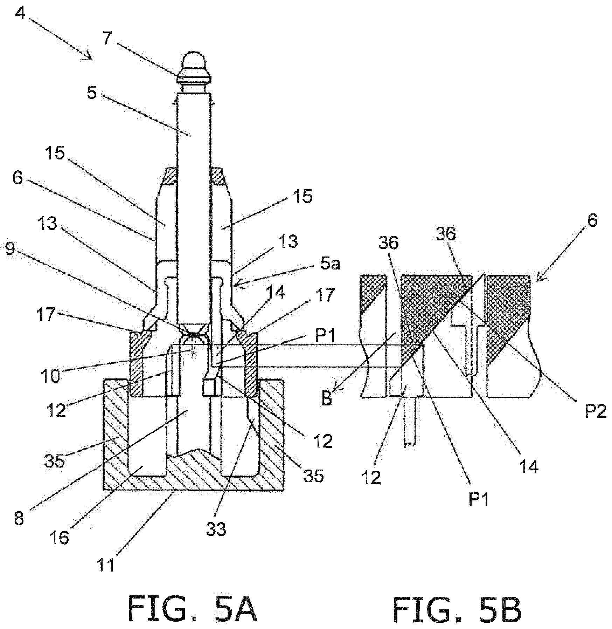

The lancet body 5 has a connector 7 that is connected to the puncture instrument 1 provided at the rear end side (second end side) of the lancet body 5, a substantially circular columnar puncture needle cover 8 that is provided on the front end side, and a breakable component 9 that is provided between the front end and the rear end. A puncture needle 10 (see FIG. 5) is embedded along the longitudinal direction of the breakable component 9. The puncture needle cover 8 is provided with a disc-shaped base portion 11 at the end on its front end side (first end side). The base portion 11 has a face that is substantially perpendicular to the puncture direction, and as shown in FIG. 3, can make the puncture needle cartridge 4 stand upright in a state in which the connector 7 is facing up.

Returning to FIG. 2, two guide protrusions 12 that protrude toward the outer peripheral direction are provided in axial symmetry to the puncture needle cover 8 on the front end side of the breakable component 9. Two slender sliding protrusions 13 that protrude toward the outer peripheral direction are provided in axial symmetry on the rear end side of the breakable component 9.

On the inner face side of the lancet case 6, two spiral guide components 14 (the broken line portions in FIG. 2) are formed from the front end side toward the rear end side.

The spiral guide components 14 slide on the guide protrusions 12 of the lancet body 5 to the front end side, which gives the lancet case 6 a rotational force in a specific rotational direction.

The "specific rotational direction" here is the direction in which the lancet case 6 rotates in conjunction with the sliding of the guide components 14 over the guide protrusions 12 to the front end side, that is, the relative motion of the lancet case 6 along the puncture direction with respect to the lancet body 5.

In this embodiment, the rotational direction is, for example, the clockwise direction when the lancet body 5 is viewed from the base portion 11 side, as shown by the C direction in FIG. 2 (hereinafter the specific rotational direction shall be called clockwise).

The two guide components 14 slide over the specific guide protrusions 12 to which they each correspond.

Accordingly, as will be described in detail below, when the lancet case 6 moves toward the base portion 11 at the front end, the lancet case 6 rotates clockwise around the lancet body 5.

The lancet case 6 has two sliding grooves 15 for guiding the sliding protrusions 13 of the lancet body 5 to the rear end side. The sliding protrusions 13 slide back and forth along the puncture direction within the engaged sliding grooves 15.

FIG. 3 is an oblique view of the unused puncture needle cartridge 4. In the unused puncture needle cartridge 4, the breakable component 9 in which the puncture needle 10 is embedded has not been broken (see FIG. 5).

The lancet case 6 is attached on the outer periphery of a middle section 5a of the lancet body 5 in a state in which the breakable component 9 is not broken. The center axis of the columnar lancet body 5 coincides with the center axis of the cylindrical lancet case 6.

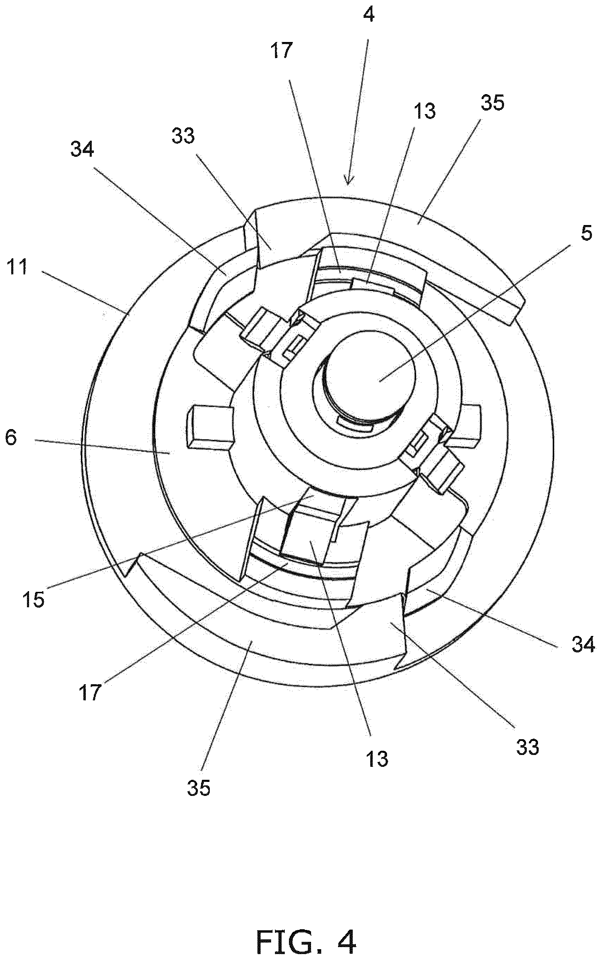

FIG. 4 is a view of the puncture needle cartridge in the state in FIG. 3, as seen obliquely from above.

FIG. 5A is a cross section along the A plane in FIG. 3.

As shown in FIG. 5A, the lancet case 6 is attached to the outer periphery of the middle section 5a of the lancet body 5. From the front end of the lancet case 6 to the base portion 11 is formed a space 16 in which the lancet case 6 moves to the base portion 11 side.

FIG. 5B is a developed view of the interior of the lancet case 6, in which the engaged state between the spiral guide component 14 and the guide protrusion 12 is shown.

Inside the lancet case 6, the guide components 14 are in contact on the front end side with the guide protrusions 12 of the puncture needle cover 8 at the sliding start position PI.

In order to form this state, the puncture needle cartridge 4 is manufactured by the following manufacturing process.

First, the lancet case 6 is inserted into the lancet body 5, and the guide components 14 on the inner face side are pressed down until they contact with the guide protrusions 12.

Furthermore, when the lancet case 6 is pushed down, the lancet case 6 rotates as the spiral guide components 14 slide along the guide protrusions 12 to the front end side. When the lancet case 6 has rotated to 30 degrees, for example, the breakable component 9 is twisted (elastic twisting that does not reach breaking).

When the lancet case 6 is then released, the lancet case 6 tries to turn backward and lift up under the restoring force of the breakable component 9, but the distal ends of the sliding protrusions 13 hold down the lancet case 6 with receivers 17.

Therefore, the elasticity of the sliding protrusions 13 causes the front end side of the guide components 14 to stop at the sliding start position PI in a state of being in contact with the guide protrusions 12. That is, at the sliding start position PI, the lancet case 6 is attached to the outer periphery of the middle section 5a of the lancet body 5.

At the time of use, as shown in FIG. 1, the puncture needle cartridge 4 is inserted into the puncture instrument 1. At this point, since the tip of the puncture instrument 1 is in contact with the receivers 17 of the lancet case 6, the lancet case 6 is pressed by the puncture instrument 1 and moves (slides) from the sliding start position PI (the middle section 5a of the lancet body 5) toward the base portion 11 at the front end.

When the lancet case 6 slides to the front end side, the lancet case 6 turns clockwise along the spiral guide components 14.

More specifically, as shown in FIG. 5B, when the lancet case 6 slides to the front end side, the guide components 14 start sliding inside the lancet case 6 from the sliding start position PI toward the front end side (the B direction in FIG. 5B), and slides over the guide protrusions 12 to the sliding end position P2.

When the spiral guide components 14 slide along the guide protrusions 12, the lancet case 6 receives a rotational force in a specific direction (such as clockwise) and rotates clockwise. The lancet case 6 then slides to the base portion 11 side on the front end side while rotating.

The rotating lancet case 6 turns the portion of the lancet body 5 that is further on the rear end side than the breakable component 9, in the C direction shown in FIG. 2 (clockwise), via the sliding grooves 15 and the sliding protrusions 13.

Meanwhile, the portion of the lancet body 5 further on the front end side than the breakable component 9 rotates counterclockwise (the D direction in FIG. 2) via the guide protrusions 12 under the reaction force from the spiral guide components 14.

As a result, a turning force in the reverse direction is generated at a connecting part before and after the breakable component 9, and the breakable component 9 can be broken.

That is, when the lancet case 6 attached to the outer periphery of the middle section 5a slides to the side of the base portion 11 provided with an operation as the puncture needle cartridge 4 is attached to the front end portion of the puncture instrument 1, a rotational force in the reverse direction is imparted to the front and rear portions of the breakable component 9. In other words, the puncture needle cartridge 4 itself has a turning mechanism for breaking the puncture needle cover 8 from the puncture needle cartridge 4 at the breakable component 9.

Therefore, the breakable component 9 can be broken without being affected by the state of attachment of the puncture needle cartridge 4 to the puncture instrument 1.

As a result, the puncture needle cartridge 4 can be properly attached to the puncture instrument 1.

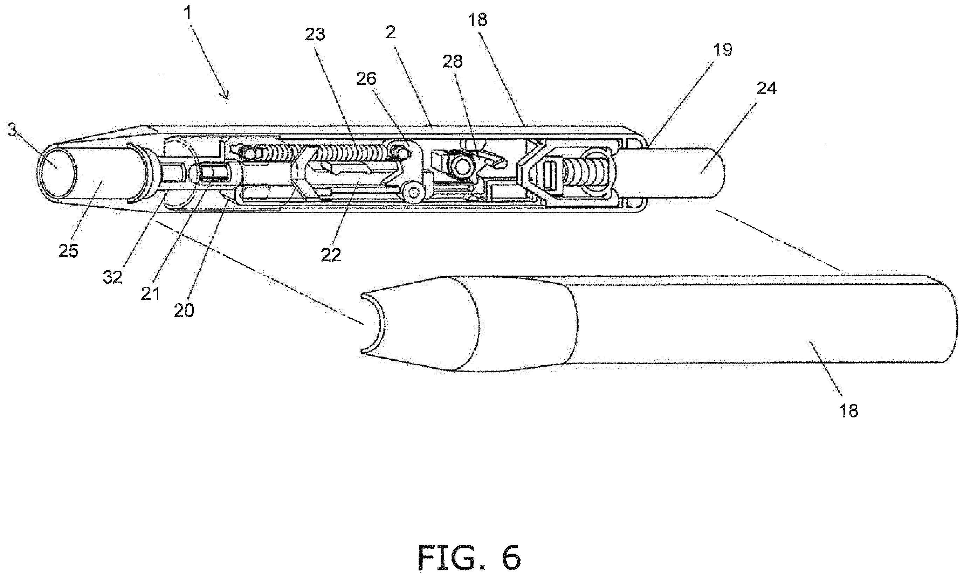

FIG. 6 is an exploded oblique view of the puncture instrument 1 to which the puncture needle cartridge 4 is attached.

The puncture instrument 1 comprises the tubular main body case 2 having the front end opening 3, a grip portion 18, and a rear end opening 19. A main rod 20 is provided in the main body case 2. An injection rod 22 having a lancet holder 21 is attached to the main rod 20 so as to be slidable in the longitudinal direction.

The main body case 2 is provided with a main spring (an example of a biasing body) 23 for biasing the injection rod 22 toward the front end side, and an operation button 24 on a rear side of the main body case 2. A tubular bushing 25 into which the puncture needle cartridge 4 is inserted is provided in the front end opening 3.

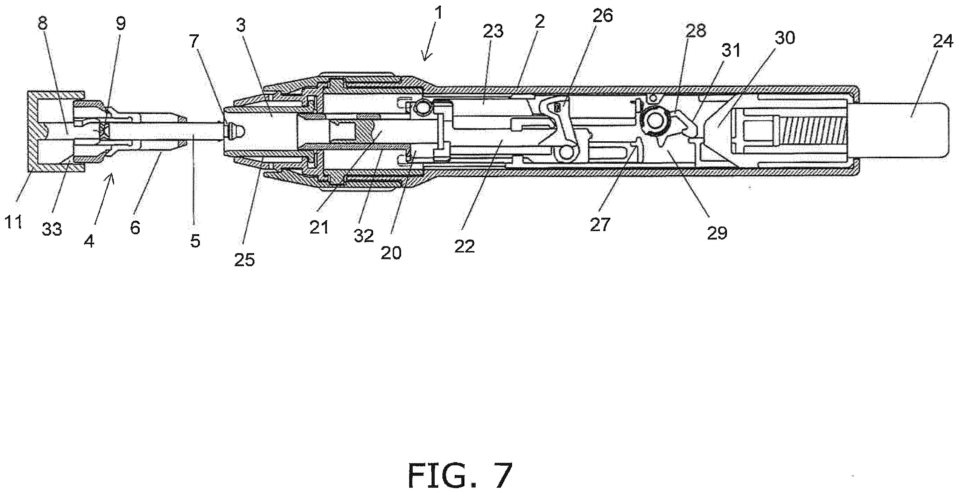

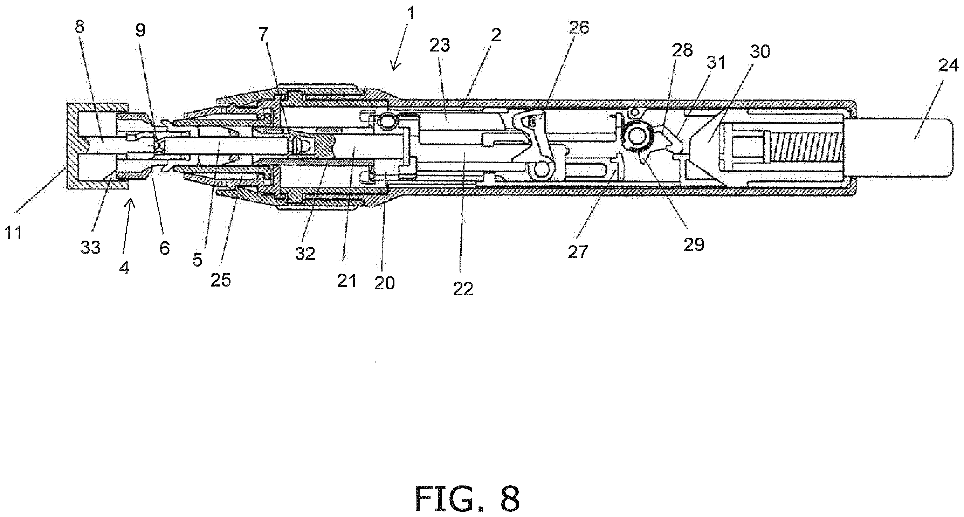

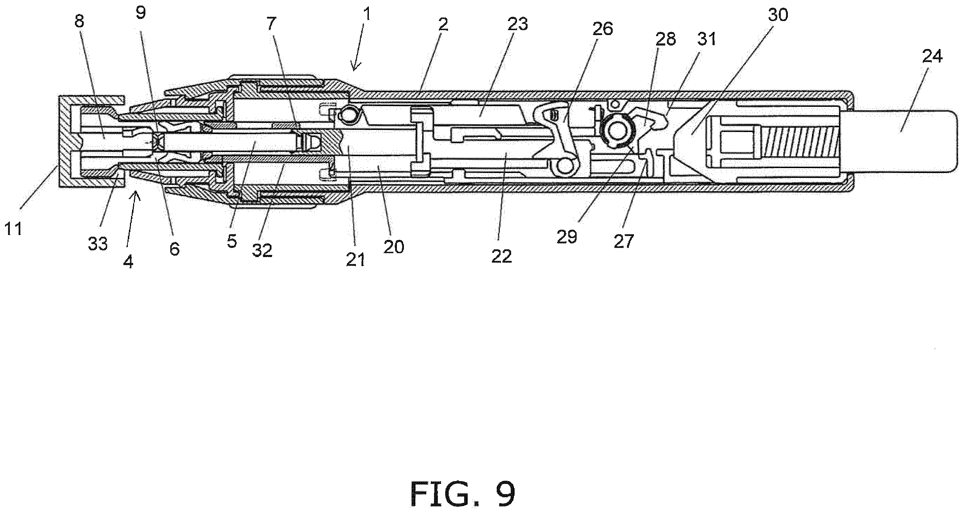

When the puncture needle cartridge 4 is attached to the puncture instrument 1, as shown in FIG. 7, the connector 7 of the puncture needle cartridge 4 is pushed into the front end opening 3 of the puncture instrument 1. As shown in FIG. 8, the connector 7 contacts with the front end of the lancet holder 21, and is attached to the lancet holder 21 as shown in FIG. 9. This attaches the puncture needle cartridge 4 to the puncture instrument 1.

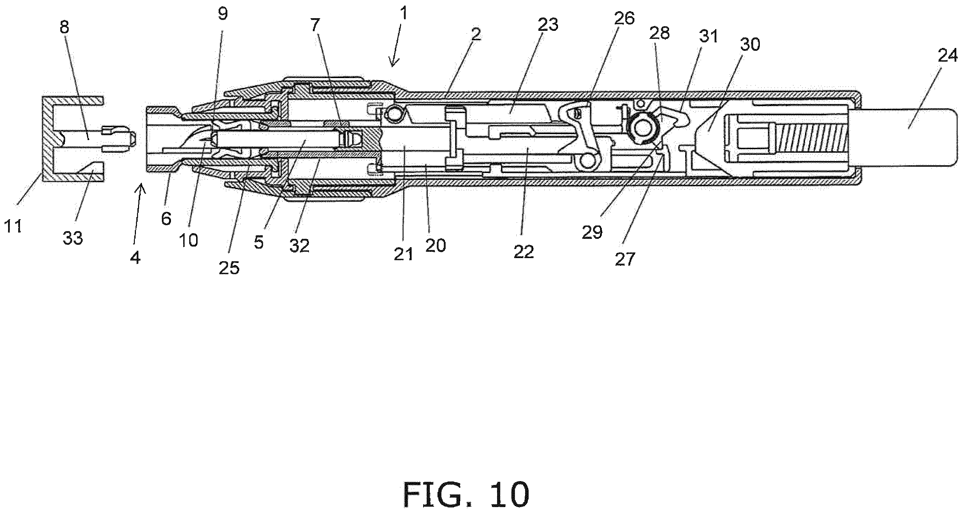

When the base portion 11 is pushed in toward the lancet case 6 side from the state in FIG. 8 to the state in FIG. 9, as discussed above, the lancet case 6 rotates clockwise while sliding to the base portion 11 side, and the breakable component 9 is broken. When the user then pulls the base portion 11, as shown in FIG. 10, the puncture needle cover 8 covering the tip of the puncture needle 10 is easily removed.

Meanwhile, in the puncture instrument 1 in FIG. 9, the lancet body 5 pushes the lancet holder 21 and the injection rod 22 to the rear end side. Consequently, the main spring 23 is pulled to the rear end side via a puncture cam 26, and the puncturing force is stored.

As shown in FIG. 10, this puncturing force is held by engaging a locking prong 27 of the injection rod 22 with an engagement prong 29 of a lock body 28 on the rear end side of the main body case 2.

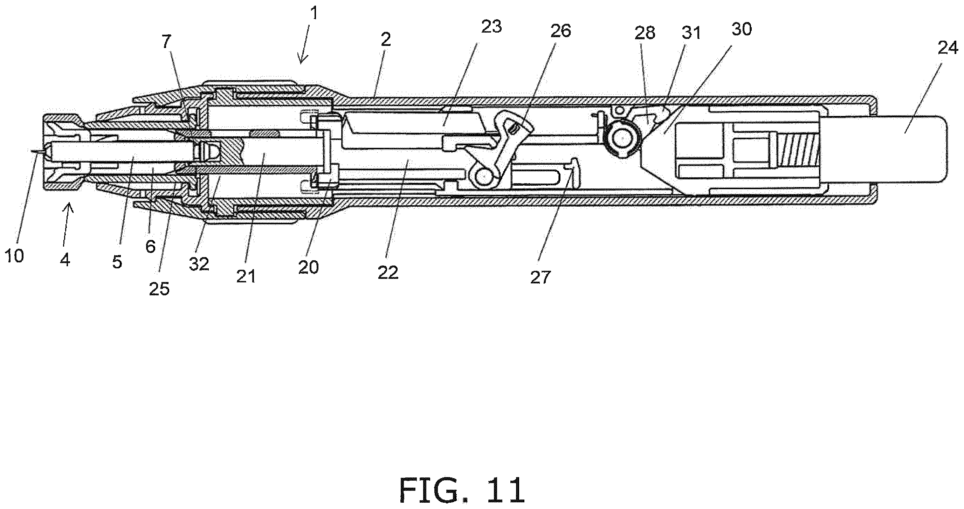

After this, when the user presses the operation button 24, an operation body 30 at the front end of the operation button 24 pushes up an ejection prong 31 as shown in FIG. 11. As a result, the lock body 28 rotates, and the locking prong 27 and the engagement prong 29 are disengaged (see FIG. 10).

The injection rod 22 then shoots out under the biasing force of the main spring 23, the puncture needle 10 is pushed out of the front end of the puncture needle cartridge 4, and a puncturing operation is performed.

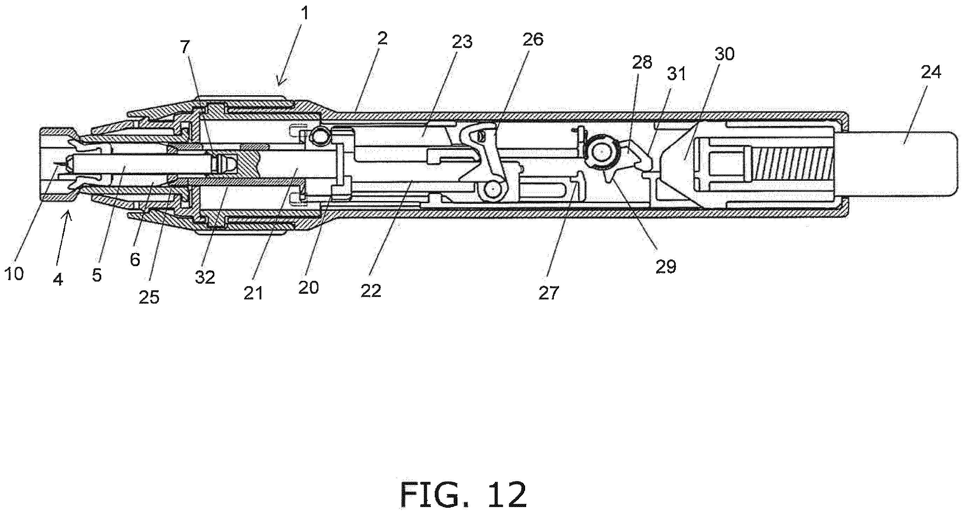

Then, as shown in FIG. 12, the main spring 23 pulls the injection rod 22 to a set position, and the puncture needle 10 is pulled back into the lancet case 6.

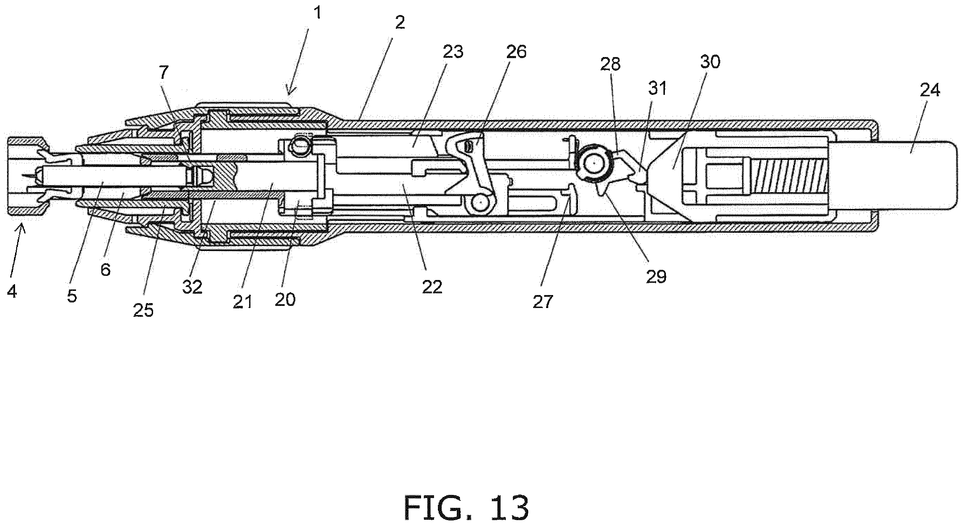

After this, when the user pushes the operation button 24, as shown in FIG. 13, a holder insertion tube 32 at the front end of the main rod 20 pushes the lancet case 6 via the operation body 30 and the lock body 28.

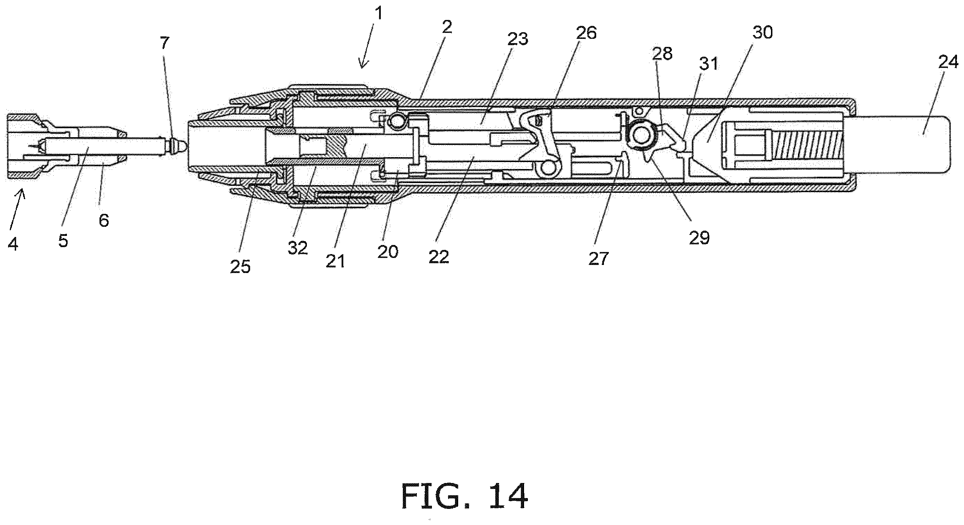

Then, as shown in FIG. 14, the connector 7 of the puncture needle cartridge 4 is removed from the lancet holder 21, and the puncture needle cartridge 4 is ejected from the puncture instrument 1.

That is, when the operation button 24 is operated, the lock body 28 switches between puncturing operation of the puncture needle cartridge 4 and ejection operation of the puncture needle cartridge 4.

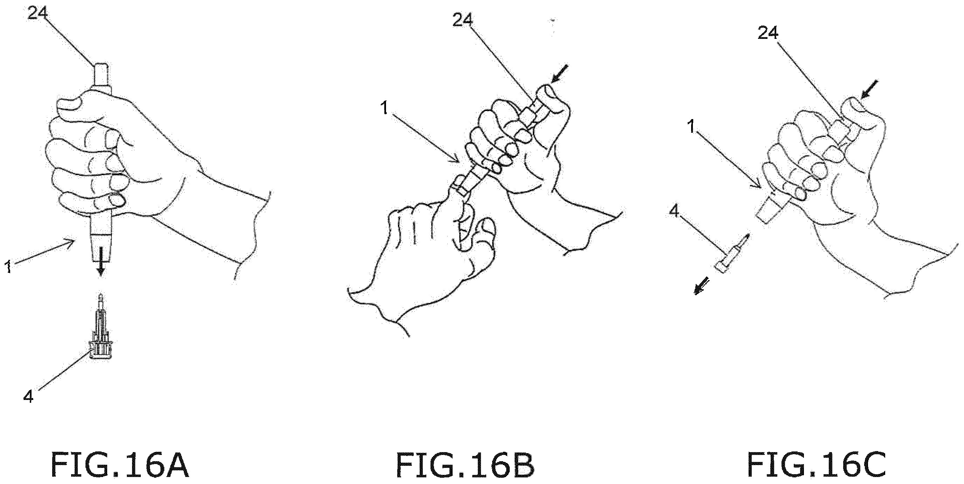

In actual use, as shown in FIG. 16A, the user grips the puncture instrument 1, and can attached the puncture needle cartridge 4 to the puncture instrument 1 merely by pushing down on the upright puncture needle cartridge 4.

After this, as shown in FIG. 16B, the user presses the operation button 24 while still gripping the puncture instrument 1, without having to change hands, thereby performing a puncture operation with the puncture needle 10.

As shown in FIG. 16C, when the user once again presses the operation button 24, the lock body 28 switches the operation of the operation button 24 from a puncture operation to a disposal operation, so the disposal operation of the puncture needle cartridge 4 is carried out.

That is, the user can perform attachment, puncture, and disposal operations without changing hands, still holding the puncture instrument 1 the same way as at first, which is more convenient.

In the above description, the basic configuration and operation of the puncture needle cartridge 4 were described.

In this embodiment, with the puncture needle cartridge 4 shown in FIG. 3, as described above, the lancet case 6 slides from the middle section 5a of the lancet body 5 to the front end side, thereby actuating the turning mechanism and rotating the lancet case 6 in a specific rotational direction (such as clockwise).

However, in actual use, the user may accidentally twist the lancet case 6 counterclockwise when handling the puncture needle cartridge 4.

If this happens, since the lancet case 6 is merely stopped at the outer periphery of the middle section 5a of the lancet body 5 by the elasticity of the sliding protrusions 13, there is the possibility that the twisting force from the user will cause the lancet case 6 to turn counterclockwise, and the lancet case 6 will end up sliding to the rear end side (that is, the opposite side from the base portion 11).

This creates the possibility that the guide components 14 will come loose from the guide protrusions 12 inside of the lancet case 6 (the guide components 14 disengage from the specific guide protrusions 12), so that the turning mechanism does not operate properly.

That is, when the lancet case 6 slides to the rear end side, as shown in FIG. 5B, in the interior of the lancet case 6 the guide components 14 slide from the sliding start position PI to the rear end side (the opposite direction from the B direction). This results in the guide components 14 disengaging from the specific guide protrusions 12.

In this state, even if the puncture needle cartridge 4 is pushed into the tip of the puncture instrument 1, the guide components 14 cannot slide on the specific guide protrusions 12 from the sliding start position PI to the sliding end position P2. The lancet case 6 therefore cannot be rotated, and as a result the breakable component 9 is not properly broken.

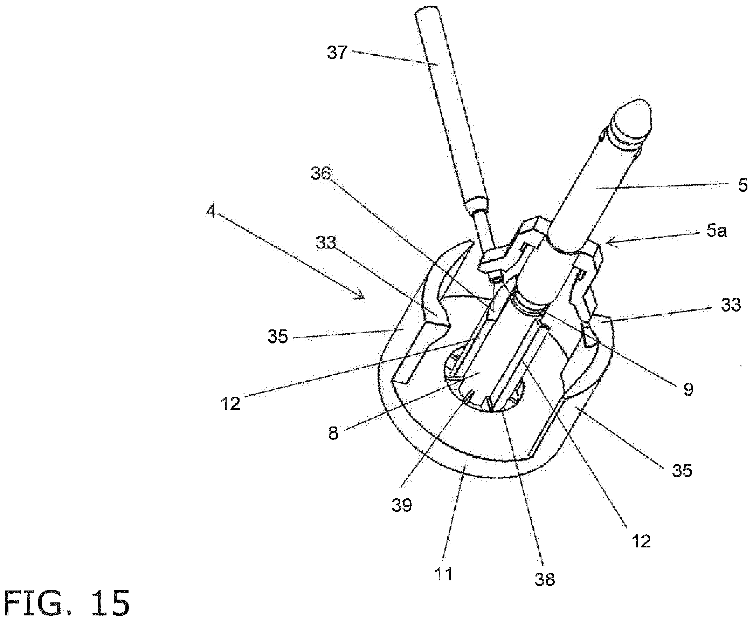

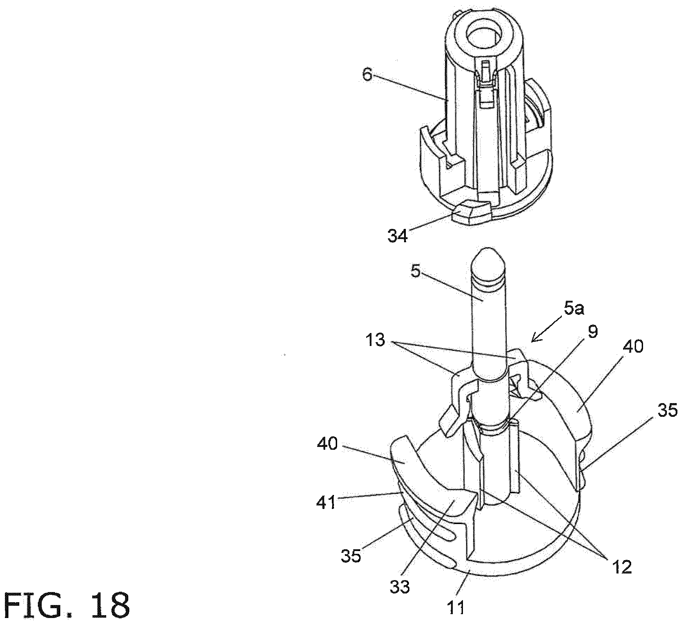

In view of this, with the puncture needle cartridge 4 of this embodiment, as shown in FIGS. 3 and 4, a pair of stoppers 33 (see FIGS. 15 and 18) for preventing the lancet case 6 from sliding from the middle section 5a (sliding start position PI) to the rear end side is provided to the puncture needle cover 8 in a state in which the lancet case 6 is attached to the outer periphery of the middle section 5a of the lancet body 5.

The stoppers 33 are in contact with the lancet case 6 so as to sandwich the outer peripheral face of the front end portion of the lancet case 6 from the outside.

Meanwhile, the lancet case 6 is provided with two engagement prongs 34 (see FIG. 18) that protrude outward at the outer peripheral face of the front end portion formed in a disc shape. The engagement prongs 34 are provided at locations corresponding to the stoppers 33.

In order to keep the lancet case 6 from sliding to the rear end side with respect to the lancet body 5, the stoppers 33 are brought into contact with the engagement prongs 34 (the stoppers 33 are engaged with the engagement prongs 34), which prevents the lancet case 6 from rotating counterclockwise.

Therefore, the lancet case 6 rotates only in the clockwise direction (the specific rotational direction).

Preventing the counterclockwise rotation of the lancet case 6 keeps the lancet case 6 from sliding to the rear end side.

Therefore, the lancet case 6 slides only to the front end side, based on the position where the stoppers 33 and the engagement prongs 34 come into contact (the initial position).

Consequently, even if the user should accidentally twist the lancet case 6 in the opposite direction (counterclockwise direction) from the specific rotational direction, the stoppers 33 will keep the lancet case 6 from rotating in the counterclockwise direction. Therefore, the lancet case 6 is restricted from sliding to the rear end side.

That is, because of the stoppers 33, the lancet case 6 is able to slide only from the initial position to the front end side. Therefore, the guide components 14 and the guide protrusions 12 are prevented from disengaging on the inner peripheral face side of the lancet case 6, and the turning mechanism can be operated properly.

As a result, the puncture needle cartridge 4 can be properly attached to the puncture instrument 1.

The force at which the stoppers 33 prevent the lancet case 6 from sliding to the rear end side is much greater than the force at which the sliding protrusions 13 hold down the lancet case 6. Therefore, sliding of the lancet case 6 to the rear end side can be properly prevented.

Furthermore, in this embodiment, as shown in FIG. 18, the engagement prongs 34 are provided on the outer peripheral portion furthest away from the center axis of the lancet case 6. By bringing the engagement prongs 34 and the stoppers 33 into contact with each other, the stoppers 33 and the engagement prongs 34 can come into contact on the outermost peripheral side of the lancet case 6.

To describe this more specifically, as shown in FIG. 3, the disc-shaped base portion 11 is provided with a pair of outer peripheral walls 35 that extend along the puncture direction toward the rear end side (the upper side in FIG. 3).

As shown in FIG. 4, the outer peripheral walls 35 are formed in an arc shape along the outer periphery, and are provided symmetrically with respect to the center axis of the puncture needle cover 8. The outer peripheral walls 35 extend to the outer periphery of the front end portion of the lancet case 6 attached to the outer periphery of the middle section 5a of the lancet body 5. The stoppers 33 are respectively provided on the inner peripheral sides of the outer peripheral walls 35. The stoppers 33 are provided at equidistant spacing with respect to the outer periphery of the lancet case 6.

Therefore, even if the user should accidentally twist the lancet case 6 counterclockwise, since the stoppers 33 are contacting with the engagement prongs 34 at the outermost peripheral portion from the center axis of the lancet case 6, counterclockwise rotation of the lancet case 6 can be easily prevented. That is, the lancet case 6 can be prevented from sliding to the rear end side in the puncture direction.

In this embodiment, when the lancet case 6 slides to the front end side in the puncture direction, the guide components 14 slide toward the front end side while rotating along the guide protrusions 12, and this obtains a clockwise rotational force of the lancet case 6. However, the present invention is not limited to this.

For example, the configuration may be such that the guide components 14 slide on the guide protrusion 12 to the front end side so that the lancet case 6 obtains a counterclockwise rotational force (opposite to the clockwise direction).

Also, in this embodiment, as shown in FIG. 15, the guide protrusions 12 provided to the puncture needle cover 8 have guide faces 36 for guiding the spiral guide components 14 (see FIG. 2) of the lancet case 6. The guide faces 36 are coated with a silicone oil, as an example of a lubricating oil.

Therefore, when the lancet case 6 slides from the state in FIG. 5A to the front end side, as described above, as shown in FIG. 5B, the guide components 14 slide within the lancet case 6 from the sliding start position P1 toward the front end side (the B direction in FIG. 5B). The guide components 14 then slide on the guide faces 36 of the guide protrusions 12 to the sliding end position P2.

At this point, since the guide faces 36 are coated with silicone oil, the frictional force generated between the guide components 14 and the guide faces 36 is reduced, allowing the lancet case 6 to be slid with less force.

As a result, the breakable component 9 can be broken with a small force.

The silicone oil applied to the guide faces 36 is a biocompatible oil and is harmless if it sticks to or is absorbed by the human body.

In addition, in order to perform the above operation, it is necessary to appropriately apply silicone oil to the guide faces 36 during manufacture. If too much silicone oil is applied, for example, there is a possibility that the silicone oil will run down from the guide faces 36 and flow out to the outer surface of the puncture needle cartridge 4. If this happens, the silicone oil will stick to the fingers of the user during use of the device, which can be unpleasant for the user.

In view of this, as shown in FIG. 15, during manufacture, first silicone oil is sprayed onto the guide faces 36 with a nozzle 37. Next, air is blown against the guide faces 36 using an air nozzle (not shown) before the silicone oil runs down from the guide faces 36.

This blows away any excess oil from the guide faces 36, leaving the guide faces 36 thinly covered with silicone oil, so no silicone oil oozes out from the guide faces 36.

As a result, it is possible to prevent the silicone oil from oozing out to the outer surface of the puncture needle cartridge 4, thereby preventing the user from feeling uncomfortable.

Also, in order to prevent the silicone oil from oozing out to the outer surface of the puncture needle cover 8, as shown in FIG. 15, the puncture needle cover 8 may be provided with a reservoir 38 for holding any silicone oil that oozes out from the guide faces 36.

More specifically, the base portion 11, which has a face that is substantially perpendicular to the puncture direction, is provided on the front end side of the puncture needle cover 8. The reservoir 38 (a recessed part) is formed in a ring shape on the face of the base portion 11 on the puncture needle cover 8 side so as to surround the puncture needle cover 8.

Therefore, even if the silicone oil flows out from the guide faces 36 as a result of spraying silicone oil onto the guide faces 36 with the nozzle 37 during manufacture, it is possible to catch and hold the silicone oil that has flowed in the reservoir 38.

As a result, the silicone oil can be prevented from oozing out onto the outer surface of the puncture needle cartridge 4, so there is no discomfort on the part of the user.

As shown in FIG. 15, a plurality of ribs 39 are provided inside the reservoir 38, radiating out from the center axis of the puncture needle cover 8. Therefore, the silicone oil can be kept in the reservoir 38 that is finely partitioned by the ribs 39, so that the reservoir 38 can properly hold the silicone oil.

Furthermore, in order to prevent the silicone oil from oozing out onto the outer surface of the puncture needle cartridge 4, the silicone oil may be held on the outer periphery of the breakable component 9.

That is, the outside diameter of the breakable component 9 of the lancet body 5 is formed smaller (weaker) than the outside diameter of the puncture needle cover 8 in order to ensure breakage. That is, a recess that reduces the outside diameter is formed around the outer periphery of the breakable component 9. Therefore, the configuration may be such that the silicone oil is held in this recess.

More specifically, during manufacture, silicone oil is sprayed onto the guide faces 36 with the nozzle 37 in a state in which the base portion 11 is placed on the upper side (a state in which the top and bottom in FIG. 15 are reversed). At this point, even if silicone oil oozes out from the guide faces 36, that silicone oil can be caught and held by the recess in the breakable component 9.

As a result, the silicone oil can be prevented from oozing out onto the outer surface of the puncture needle cartridge 4, so there is no discomfort on the part of the user.

A silicone oil that is the same as the silicone oil applied to the guide faces 36 is applied in advance as a lubricating oil to the puncture needle 10. Therefore, when the puncture needle cover 8 is removed from the puncture needle 10, even if some of the silicone oil held in the breakable component 9 sticks to the puncture needle 10 and then sticks to or is absorbed by the human body, there will be no problem since the oil is a harmless, biocompatible oil.

Here, when using a puncture needle cartridge 4 manufactured as above, in actual use there will be times when the user grabs the base portion 11 of the puncture needle cartridge 4 and attaches the puncture needle cartridge 4 to the puncture instrument 1. When this happens, the user may inadvertently stick the puncture needle cartridge 4 into the puncture instrument 1 at an angle.

In such a situation, if the rear end portion of the lancet case 6 obliquely comes into contact with the front end opening 3 of the puncture instrument 1 from the state shown in FIG. 1, for example, then an oblique force will be exerted on the rear end portion of the lancet body 5 via the lancet case 6. At this point, there is a risk that the lancet body 5 will be bent by the breakable component 9 inside the lancet case 6 shown in FIG. 5. Consequently, there is a possibility that the guide components 14 will come loose from the guide protrusions 12, and the turning mechanism will not operate.

In view of this, with the puncture needle cartridge 4 in this embodiment, as shown in FIGS. 3 and 4, support components (the stoppers 33 and the outer peripheral walls 35) that support the outer periphery of the lancet case 6 are provided to the base portion 11 of the puncture needle cover 8. That is, support components are constituted by the stoppers 33 and the outer peripheral walls 35 of the puncture needle cover 8.

More specifically, the outer peripheral walls 35 and the stoppers 33 provided to the outer peripheral walls 35 contact with the outer periphery of the front end portion of the lancet case 6, and provide support so as to sandwich the outer peripheral face of the lancet case 6 from opposing sides.

Therefore, as shown in FIG. 3, this results in a state in which the lancet case 6, the stoppers 33, the outer peripheral walls 35, and the base portion 11 are integrated. That is, a state is formed in which the outer peripheral portion of the puncture needle cartridge 4 is integrated.

Therefore, even if the puncture needle cartridge 4 is stuck obliquely into the puncture instrument 1, since the outer peripheral part of the puncture needle cartridge 4 is held by the support components, the lancet body 5 inside the lancet case 6 can be protected. This prevents the lancet body 5 from being bent by the breakable component 9.

As a result, the guide components 14 can be prevented from coming loose from the guide protrusions 12, and the turning mechanism can be operated properly. Thus, the puncture needle cartridge 4 can be properly attached to the puncture instrument 1.

As described above, the stoppers 33 in this embodiment prevent the lancet case 6 from rotating in the opposite direction from the specific rotational direction, which prevents the lancet case 6 from sliding from the middle section 5a to the rear end side, and also supports the lancet case 6 from the outer peripheral face side.

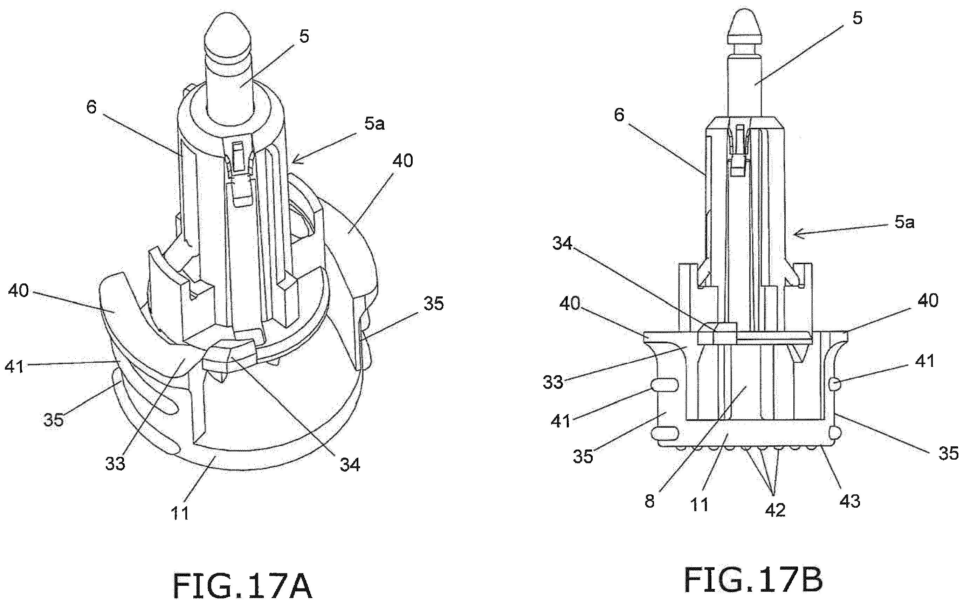

Here, as shown in FIGS. 17A and 17B, finger rests (first orientation holder) 40 that protrude outward in an arc shape may be provided to the ends of the outer peripheral walls 35 of the puncture needle cover 8 on the rear end side (the upper side in FIG. 17).

With this configuration, when the user grasps the outer peripheral walls 35 of the puncture needle cover 8 with his fingers, those fingers come into contact with the finger rests 40 on the rear end side of the outer peripheral walls 35, so the user can apply force to the finger rests 40 and push the puncture needle cartridge 4 into the puncture instrument 1.

At this point, when the user grasps the outer peripheral walls 35, the grasping force from the user is transmitted to the lancet case 6 through the stoppers 33 provided on the inner peripheral face side of the outer peripheral walls 35. That is, the user is indirectly grasping the lancet case 6, so the lancet case 6 can be stably supported.

As a result, the user can attach the puncture needle cartridge 4 to the puncture instrument 1 while indirectly grasping the lancet case 6.

Furthermore, a plurality of anti-slip components 41 that protrude outward in an arc shape may be provided to the outer peripheral faces of the outer peripheral walls 35 so that they can be gripped by the user's fingers.

With this configuration, the user can grip the outer peripheral faces of the outer peripheral walls 35 by a natural operation while looking at the anti-slip components 41. Also, the texturing of the anti-slip components 41 affords a firm grip on the outer peripheral walls 35.

Furthermore, a finger rest 43 (second orientation holder) that has a plurality of protrusions 42 protruding on the front end side (puncture side) may be formed on the face on the front end side of the base portion 11 (the lower side in FIG. 17B).

With this configuration, when the puncture needle cover 8 is grasped and attached to the puncture instrument 1, the user can place his fingers on the finger rest 43 provided to the front end face and push the puncture needle cartridge 4 into the puncture instrument 1 at the end of the attachment operation.

As a result, the puncture needle cartridge 4 can be properly attached to the puncture instrument 1.

As described above, the puncture needle cartridge 4 of this embodiment comprises the lancet body 5 and the tubular lancet case 6 attached to the outer periphery of the lancet body 5. The lancet body 5 has the puncture needle 10 embedded in the breakable component 9 of the lancet body 5 in the puncture direction, the guide protrusions 12 provided to the puncture needle cover 8 on the front end side of the breakable component 9, and the sliding protrusions 13 provided more on the rear end side than the breakable component 9. The lancet case 6 has the spiral guide components 14 that are formed from the front end side toward the rear end side and allow the lancet case 6 to obtain the rotational force from the guide protrusions 12, and the sliding grooves 15 that guide the sliding protrusions 13. The lancet case 6 is attached to the outer periphery of the middle section 5a of the lancet body 5 in a state in which the breakable component 9 is not broken.

That is, when the lancet case 6 attached to the outer periphery of the middle section 5a is slid to the front end side along with the operation of attaching the puncture needle cartridge 4 to the puncture instrument 1, a rotational force is imparted in opposite directions to the front end side and rear end side of the breakable component 9. That is, the puncture needle cartridge 4 itself has a turning mechanism for breaking the breakable component 9. Therefore, it is possible to stably break the breakable component 9 by actuating the turning mechanism without being affected by the state of attachment of the puncture needle cartridge 4 to the puncture instrument 1.

In this embodiment, in order to operate the turning mechanism properly, the puncture needle cover 8 is provided with the stoppers 33 for restricting the sliding of the lancet case 6 from the middle section 5a to the rear end side (counterclockwise rotation).

Accordingly, the lancet case 6 slides only from the middle section 5a to the front end side, so the lancet case 6 rotates only in a specific direction. Therefore, breakable component 9 can be stably broken by appropriately actuating the turning mechanism.

As a result, the puncture needle cartridge can be properly attached to the puncture instrument.

### INDUSTRIAL APPLICABILITY

The present invention is expected to find broad application as a puncture needle cartridge for puncturing a human body with a needle and allowing blood to flow out in order to measure a blood glucose level, for example, as well as a puncture instrument to which this puncture needle cartridge is attached.

### REFERENCE SIGNS LIST

- 1: puncture instrument
- 2: main body case
- 3: front end opening
- 4: puncture needle cartridge
- 5: lancet body
- 5a: middle section
- 6: lancet case
- 7: connector
- 8: puncture needle cover
- 9: breakable component
- 10: puncture needle
- 11: base portion
- 12: guide protrusion
- 13: sliding protrusion
- 14: guide component
- 15: sliding groove
- 16: space
- 17: receiver
- 18: grip portion
- 19: rear end opening
- 20: main rod
- 21: lancet holder
- 22: injection rod
- 23: main spring
- 24: operation button
- 25: bushing
- 26: puncture cam
- 27: locking prong
- 28: lock body
- 29: engagement prong
- 30: operation body
- 31: ejection prong
- 32: holder insertion tube
- 33: stopper
- 34: engagement prong
- 35: outer peripheral wall
- 36: guide face
- 37: nozzle
- 38: reservoir
- 39: rib
- 40: finger rest (first orientation holder)
- 41: anti-slip component
- 42: protrusion
- 43: finger rest (second orientation holder)
- PI: sliding start position
- P2: sliding end position

## Claims

1. A puncture needle cartridge (4) that is attached to a puncture instrument (1), comprising:
a columnar lancet body (5) having a puncture needle (10), a first end and a second end; a connector (7) that is connected to the puncture instrument (1) on a side of the second end, which is an opposite side from the first end at which puncture is performed with the puncture needle (10); a puncture needle cover (8) that is provided on a side of the first end and covers a tip portion of the puncture needle (10) before use; a breakable component (9) that is provided to a middle section between the first end and the second end, is broken when performing puncturing, and separates the puncture needle cover (8) and exposes the tip portion of the puncture needle (10); guide protrusions (12) that are provided to the outer peripheral face of the puncture needle cover (8) on the side of the first end of the breakable component (9); and sliding protrusions (13) that are provided closer to the side of the second end than the breakable component (9); and
a tubular lancet case (6) having spiral guide components (14) formed from the side of the first end toward the side of the second end, configured to impart a rotational force in a specific direction when there is relative movement in a puncture direction in a state in which the guide protrusions (12) are engaged; and a sliding groove (15) configured to guide movement in the puncture direction in a state in which the sliding protrusions (13) are engaged, the lancet case (6) being mounted to an outer periphery of the middle section of the lancet body (5) in a state in which the breakable component (9) has not been broken,
the puncture needle cover (8) further has stoppers (33) configured to restrict sliding to the side of the second end from a state in which the lancet case (6) is mounted to the middle section.

2. The puncture needle cartridge according to Claim 1,
wherein the stoppers (33) restrict a rotation of the lancet case (6) to an opposite side from said specific direction, with respect to the lancet body (5).

3. The puncture needle cartridge according to Claim 1 or 2,
wherein the stoppers (33) are provided to an inner peripheral face of the puncture needle cover (8) and protrudes toward an outer peripheral face of the lancet case (6).

4. The puncture needle cartridge according to Claim 3,
wherein the stoppers (33) contact with an outer peripheral part of the lancet case in a state in which the lancet case (6) is mounted on the outer periphery of the middle section of the lancet body (5).

5. The puncture needle cartridge according to Claim 4,
wherein the lancet case (6) further has an engagement prong (34) that is provided to the outer peripheral part and contacts with the stoppers (33).

6. The puncture needle cartridge according to Claim 5,
wherein the engagement prong (34) is provided on an outermost peripheral side of the lancet case (6).

7. The puncture needle cartridge according to any of Claims 1 to 6,
wherein the puncture needle cover (8) further has a base portion (11) that is provided to the first end and includes a face that intersects the puncture direction, and an outer peripheral wall (35) that is provided to an outer peripheral part of the base portion (11) and is disposed opposite an outer peripheral face of the lancet case (6) along the puncture direction.

8. The puncture needle cartridge according to Claim 7,
wherein the stoppers (33) are provided on an inner peripheral face side of the outer peripheral wall (35).

9. The puncture needle cartridge according to any of Claims 1 to 8,
wherein the guide protrusions (12) have guide faces configured to guide the guide components (14) of the lancet case (6), and
a lubricating oil is applied to the guide face.

10. The puncture needle cartridge according to any of Claims 1 to 6,
wherein the guide protrusions (12) have guide faces configured to guide the guide components (14) of the lancet case (6),
a lubricating oil is applied to the guide face, and
the lancet body (5) further has a reservoir (38) configured to hold the lubricating oil.

11. The puncture needle cartridge according to Claim 10,
wherein the puncture needle cover (8) further has a base portion (11) that is provided to the first end and includes a face that intersects the puncture direction, and
the reservoir (38) is formed in a ring shape surrounding the puncture needle cover (8) on the base portion (11).

12. The puncture needle cartridge according to Claim 10 or 11,
wherein the reservoir (38) is a recessed portion formed in a face of the base portion (11) on the side of the second end.

13. The puncture needle cartridge according to any of Claims 9 to 12,
wherein the lubricating oil is held around an outer periphery of the breakable component (9).

14. The puncture needle cartridge according to any of Claims 9 to 13,
the lubricating oil applied to the guide face is applied to the puncture needle (10).

15. The puncture needle cartridge according to Claim 7,
further comprising a first orientation holder (40) that is provided to the outer peripheral face of the outer peripheral wall (35) and protrudes outward.

16. The puncture needle cartridge according to Claim 15,
further comprising an anti-slip component (41) that protrudes outward from the outer peripheral face of the outer peripheral wall (35).

17. The puncture needle cartridge according to Claim 7,
further comprising a second orientation holder (43) that is provided to a face on the side of the first end of the puncture needle cover (8) and protrudes toward the side of the first end.

18. A puncture instrument (1) to which the puncture needle cartridge (4) according to any of Claims 1 to 17 is attached, the puncture instrument comprising:
a tubular main body case (2) that has a front end opening (3) and a rear end opening (19);
a main rod (20) that is provided inside the main body case (2);
a injection rod (22) that is attached to the main rod (20) and has a lancet holder (21) to which the puncture needle cartridge (4) is attached;
a biasing body (23) configured to bias the injection rod toward a side of the front end opening (3) of the main body case (2);
an operation button (24) slidably provided at the rear end opening (19) of the main body case (2); and
a lock (28) configured to be operated with the operation button (24) and switch between a puncture operation of the puncture needle cartridge (4) and a disposal operation of the puncture needle cartridge (4).

## Patentansprüche

1. Punktionskanülenpatrone (4), die an einem Punktionsinstrument (1) befestigt ist,
umfassend:
einen säulenförmigen Lanzettenkörper (5) mit einer Punktionskanüle (10), ein erstes Ende und ein zweites Ende; einen Verbinder (7), der mit dem Punktionsinstrument (1) an einer Seite des zweiten Endes verbunden ist, die eine dem ersten Ende gegenüberliegende Seite ist, an der die Punktion mit der Punktionskanüle (10) durchgeführt wird; eine Punktionskanülenabdeckung (8), die auf einer Seite des ersten Endes bereitgestellt ist und vor der Verwendung einen Spitzenabschnitt der Punktionskanüle (10) abdeckt; eine zerbrechliche Komponente (9), die an einem Mittelteil zwischen dem ersten Ende und dem zweiten Ende vorhanden ist, die bei der Durchführung der Punktion zerbrochen wird und die Punktionskanülenabdeckung (8) trennt und den Spitzenabschnitt der Punktionskanüle (10) freilegt; Führungsvorsprünge (12), die an der Außenumfangsfläche der Punktionskanülenabdeckung (8) an der Seite des ersten Endes der zerbrechlichen Komponente (9) bereitgestellt sind; und Schiebevorsprünge (13), die näher an der Seite des zweiten Endes vorhanden sind, als die zerbrechliche Komponente (9); und
eine tubusförmige Lanzettenhülse (6) mit spiralförmigen Führungskomponenten (14), die von der Seite des ersten Endes zur Seite des zweiten Endes hin ausgebildet und so konfiguriert sind, dass sie eine Drehkraft in einer spezifischen Richtung ausüben, wenn eine relative Bewegung in einer Punktionsrichtung in einem Zustand stattfindet, in dem die Führungsvorsprünge (12) in Eingriff stehen; und eine Schiebenut (15), die so konfiguriert ist, dass sie in einem Zustand, in dem die Schiebevorsprünge (13) in Eingriff sind, eine Bewegung in der Punktionsrichtung führt, wobei die Lanzettenhülse (6) in einem Zustand, in dem die zerbrechliche Komponente (9) nicht zerbrochen ist, an einem Außenumfang des mittleren Abschnitts des Lanzettenkörpers (5) angebracht ist,
die Punktionskanülenabdeckung (8), die ferner Stopper (33) aufweist, die so konfiguriert sind, dass sie das Verschieben zur Seite des zweiten Endes von einem Zustand aus einschränken, in dem die Lanzettenhülse (6) am Mittelteil angebracht ist.

2. Punktionskanülenpatrone nach Anspruch 1,
wobei die Stopper (33) eine Drehung der Lanzettenhülse (6)
aus der spezifischen Richtung in Bezug auf den Lanzettenkörper (5) zu einer gegenüberliegenden Seite beschränken.

3. Punktionskanülenpatrone nach Anspruch 1 oder 2,
wobei die Stopper (33) an einer Innenumfangsfläche der Punktionskanülenabdeckung (8) bereitgestellt sind und
zu einer äußeren Umfangsfläche der Lanzettenhülse (6) hin herausragen.

4. Punktionskanülenpatrone nach Anspruch 3,
wobei die Stopper (33) einen Außenumfangsteil der Lanzettenhülse in einem Zustand berühren, in dem die Lanzettenhülse (6) auf den Außenumfang des mittleren Abschnitts des Lanzettenkörpers (5) montiert ist.

5. Punktionskanülenpatrone nach Anspruch 4,
wobei die Lanzettenhülse (6) ferner einen Eingriffszapfen (34) aufweist, der am Außenumfangsteil vorhanden ist und die Stopper (33) berührt.

6. Punktionskanülenpatrone nach Anspruch 5,
wobei der Eingriffszapfen (34) an einer äußersten Umfangsfläche der Lanzettenhülse (6) bereitgestellt ist.

7. Punktionskanülenpatrone nach einem beliebigen der vorstehenden Ansprüche 1 bis 6,
wobei die Punktionskanülenabdeckung (8) ferner einen Basisabschnitt (11) hat, der am ersten Ende bereitgestellt ist und eine Fläche aufweist, die die Punktionsrichtung schneidet, und eine Außenumfangswand (35), die an einem Außenumfangsteil des Basisabschnitts (11) bereitgestellt und gegenüber einer Außenumfangsfläche der Lanzettenhülse (6) entlang der Punktionsrichtung angeordnet ist.

8. Punktionskanülenpatrone nach Anspruch 7,
wobei die Stopper (33) an einer Innenumfangsflächenseite der Außenumfangswand (35) bereitgestellt sind.

9. Punktionskanülenpatrone nach einem beliebigen der vorstehenden Ansprüche 1 bis 8,
wobei die Führungsvorsprünge (12) Führungsflächen haben, die so konfiguriert sind, dass sie die Führungskomponenten (14) der Lanzettenhülse (6) führen und
ein Schmieröl auf die Führungsfläche aufgetragen wird.

10. Punktionskanülenpatrone nach einem beliebigen der vorstehenden Ansprüche 1 bis 6,
wobei die Führungsvorsprünge (12) Führungsflächen haben, die so konfiguriert sind, dass sie die Führungskomponenten (14) der Lanzettenhülse (6) führen,
ein Schmieröl auf die Führungsfläche aufgetragen wird und der Lanzettenkörper (5) ferner einen Behälter (38) hat, der so konfiguriert ist, dass et das Schmieröl speichert.

11. Punktionskanülenpatrone nach Anspruch 10,
wobei die Punktionskanülenabdeckung (8) ferner einen Basisabschnitt (11) aufweist, der am ersten Ende bereitgestellt ist und eine Fläche beinhaltet, die die Punktionsrichtung schneidet, und
der Behälter (38) in einer Ringform ausgebildet ist, die die Punktionskanülenabdeckung (8) am Basisabschnitt (11) umgibt.

12. Punktionskanülenpatrone nach Anspruch 10 oder 11,
wobei der Behälter (38) ein vertiefter Abschnitt ist, der in einer Fläche des Basisabschnitts (11) an der Seite des zweiten Endes ausgebildet ist.

13. Punktionskanülenpatrone nach einem beliebigen der vorstehenden Ansprüche 9 bis 12,
wobei das Schmieröl um einen Außenumfang der zerbrechlichen Komponente (9) herum gehalten wird.

14. Punktionskanülenpatrone nach einem beliebigen der vorstehenden Ansprüche 9 bis 13, wobei das auf die Führungsfläche aufgetragene Schmieröl auf die Punktionskanüle (10) aufgetragen wird.

15. Punktionskanülenpatrone nach Anspruch 7,
ferner umfassend einen ersten Ausrichtungshalter (40), der an der Außenumfangsfläche der Außenumfangswand (35) vorhanden ist und herausragt.

16. Punktionskanülenpatrone nach Anspruch 15,
ferner umfassend eine rutschhemmende Komponente (41), die von der Außenumfangsfläche der Außenumfangswand (35) herausragt.

17. Punktionskanülenpatrone nach Anspruch 7,
ferner umfassend einen zweiten Ausrichtungshalter (43), der an einer Fläche an der Seite des ersten Endes der Punktionskanülenabdeckung (8) bereitgestellt ist und zur Seite des ersten Endes herausragt.

18. Punktionsinstrument (1), an dem die Punktionskanülenpatrone (4) nach einem beliebigen der vorstehenden Ansprüche 1 bis 17 befestigt ist, das Punktionsinstrument umfassend:
eine tubusförmige Hauptkörperhülse (2), die eine Öffnung (3) am vorderen Ende und eine Öffnung am hinteren Ende (19) aufweist;
einen Hauptstab (20), der in der Hauptkörperhülse (2) vorhanden ist;
einen Injektionsstab (22), der am Hauptstab (20) befestigt ist und einen Lanzettenhalter (21) aufweist, an dem die Punktionskanülen-Patrone (4) befestigt ist;
einen Lenkkörper (23), der so konfiguriert ist, dass er den Injektionsstab zu einer Seite der Öffnung (3) am vorderen Ende der Hauptkörperhülse (2) lenkt;
einen Betätigungsknopf (24), der verschiebbar an der Öffnung am hinteren Ende (19) der Hauptkörperhülse (2) vorhanden ist; und
eine Sperre (28), die so konfiguriert ist, dass sie mit dem Betätigungsknopf (24) betätigt werden kann und zwischen einem Punktionsvorgang der Punktionskanülenpatrone (4) und einem Entsorgungsvorgang der Punktionskanülenpatrone (4) umschaltet.

## Revendications

1. Cartouche d'aiguille à ponction (4) qui est fixée à un instrument de ponction (1) comprenant :
un corps de lancette colonnaire (5) ayant une aiguille à ponction (10), une première extrémité et une seconde extrémité ; un connecteur (7) qui est raccordé à l'instrument de ponction (1) d'un côté de la seconde extrémité qui est un côté opposé à la première extrémité à laquelle la ponction est réalisée avec l'aiguille à ponction (10) ; un couvercle d'aiguille à ponction (8) qui est prévu d'un côté de la première extrémité et recouvre une partie de pointe de l'aiguille à ponction (10) avant l'utilisation ; un composant cassable (9) qui est prévu sur une section centrale entre la première extrémité et la seconde extrémité, est cassé lors de la réalisation de la ponction, et sépare le couvercle d'aiguille à ponction (8) et expose la partie de pointe de l'aiguille à ponction (10) ; des saillies de guidage (12) qui sont prévues sur la face périphérique externe du couvercle d'aiguille à ponction (8) sur le côté de la première extrémité du composant cassable (9) ; et des saillies coulissantes (13) qui sont prévues plus à proximité du côté de la seconde extrémité que le composant cassable (9) ; et
un étui de lancette tubulaire (6) ayant des composants de guidage en spirale (14) formés à partir du côté de la première extrémité vers le côté de la seconde extrémité, configuré pour communiquer une force de rotation dans une direction spécifique lorsqu'il existe un mouvement relatif dans une direction de ponction dans un état dans lequel les saillies de guidage (12) sont mises en prise ; et une rainure de coulissement (15) configurée pour guider le mouvement dans la direction de ponction dans un état dans lequel les saillies coulissantes (13) sont mises en prise, l'étui de lancette (6) étant monté sur une périphérie externe de la section centrale du corps de lancette (5) dans un état dans lequel le composant cassable (9) n'a pas été cassé,
le couvercle d'aiguille à ponction (8) a en outre des butées (33) configurées pour limiter le coulissement du côté de la seconde extrémité à partir d'un état dans lequel l'étui de lancette (6) est monté sur la section centrale.

2. Cartouche d'aiguille à ponction selon la revendication 1,
dans laquelle les butées (33) limitent une rotation de l'étui de lancette (6) à un côté opposé de ladite direction spécifique, par rapport au corps de lancette (5).

3. Cartouche d'aiguille à ponction selon la revendication 1 ou 2,
dans laquelle les butées (33) sont prévues sur une face périphérique interne du couvercle d'aiguille à ponction (8) et font saillie vers une face périphérique externe de l'étui de lancette (6).

4. Cartouche d'aiguille à ponction selon la revendication 3,
dans laquelle les butées (33) sont en contact avec une partie périphérique externe de l'étui de lancette dans un état dans lequel l'étui de lancette (6) est monté sur la périphérie externe de la section centrale du corps de lancette (5).

5. Cartouche d'aiguille à ponction selon la revendication 4,
dans laquelle l'étui de lancette (6) a en outre une dent de mise en prise (34) qui est prévue sur la partie périphérique externe et est en contact avec les butées (33).

6. Cartouche d'aiguille à ponction selon la revendication 5,
dans laquelle la dent de mise en prise (34) est prévue sur un côté périphérique situé le plus à l'extérieur de l'étui de lancette (6).

7. Cartouche d'aiguille à ponction selon l'une quelconque des revendications 1 à 6,
dans laquelle le couvercle d'aiguille à ponction (8) a en outre une partie de base (11) qui est prévue sur la première extrémité et comprend une face qui coupe la direction de ponction, et une paroi périphérique externe (35) qui est prévue sur une partie périphérique externe de la partie de base (11) et est disposée à l'opposé d'une face périphérique externe de l'étui de lancette (6) le long de la direction de ponction.

8. Cartouche d'aiguille à ponction selon la revendication 7,
dans laquelle les butées (33) sont prévues d'un côté de la face périphérique interne de la paroi périphérique externe (35).

9. Cartouche d'aiguille à ponction selon l'une quelconque des revendications 1 à 8,
dans laquelle les saillies de guidage (12) ont des faces de guidage configurées pour guider les composants de guidage (14) de l'étui de lancette (6), et
une huile de lubrification est appliquée sur la face de guidage.

10. Cartouche d'aiguille à ponction selon l'une quelconque des revendications 1 à 6,
dans laquelle les saillies de guidage (12) ont des faces de guidage configurées pour guider les composants de guidage (14) de l'étui de lancette (6),
une huile de lubrification est appliquée sur la face de guidage, et
le corps de lancette (5) a en outre un réservoir (38) configuré pour contenir l'huile de lubrification.

11. Cartouche d'aiguille à ponction selon la revendication 10,
dans laquelle le couvercle d'aiguille à ponction (8) a en outre une partie de base (11) qui est prévue sur la première extrémité et comprend une face qui coupe la direction de ponction, et
le réservoir (38) est formé selon une forme annulaire entourant le couvercle d'aiguille à ponction (8) sur la partie de base (11).

12. Cartouche d'aiguille à ponction selon la revendication 10 ou 11,
dans laquelle le réservoir (38) est une partie évidée formée dans une face de la partie de base (11) sur le côté de la seconde extrémité.

13. Cartouche d'aiguille à ponction selon l'une quelconque des revendications 9 à 12,
dans laquelle l'huile de lubrification est maintenue autour d'une périphérie externe du composant cassable (9).

14. Cartouche d'aiguille à ponction selon l'une quelconque des revendications 9 à 13,
l'huile de lubrification appliquée sur la face de guidage est appliquée sur l'aiguille à ponction (10).

15. Cartouche d'aiguille à ponction selon la revendication 7,
comprenant en outre un premier support d'orientation (40) qui est prévu sur la face périphérique externe de la paroi périphérique externe (35) et fait saillie vers l'extérieur.

16. Cartouche d'aiguille à ponction selon la revendication 15,
comprenant en outre un composant antiglisse (41) qui fait saillie vers l'extérieur à partir de la face périphérique externe de la paroi périphérique externe (35).

17. Cartouche d'aiguille à ponction selon la revendication 7,
comprenant en outre un second support d'orientation (43) qui est prévu sur une face sur le côté de la première extrémité du couvercle d'aiguille à ponction (8) et fait saillie vers le côté de la première extrémité.

18. Instrument de ponction (1) sur lequel la cartouche d'aiguille à ponction (4) selon l'une quelconque des revendications 1 à 17 est fixée, l'instrument de ponction comprenant :
un étui de corps principal tubulaire (2) qui a une ouverture d'extrémité avant (3) et une ouverture d'extrémité arrière (19) ;
une tige principale (20) qui est prévue à l'intérieur de l'étui de corps principal (2) ;
une tige d'injection (22) qui est fixée à la tige principale (20) et a un support de lancette (21) auquel la cartouche d'aiguille à ponction (4) est fixée ;
un corps de sollicitation (23) configuré pour solliciter la tige d'injection vers un côté de l'ouverture d'extrémité avant (3) de l'étui de corps principal (2) ;
un bouton d'actionnement (24) prévu, de manière coulissante, au niveau de l'ouverture d'extrémité arrière (19) de l'étui de corps principal (2) ; et
un verrouillage (28) configuré pour être actionné avec le bouton d'actionnement (24) et effectuer une commutation entre une opération de ponction de la cartouche d'aiguille à ponction (4) et une opération de rebut de la cartouche d'aiguille à ponction (4).
